# EUROPEAN PATENT APPLICATION

(11) **EP 4 427 665 A1**
(43) Date of publication of application: **11.09.2024**
(21) Application number: 22890131.0
(22) Date of filing: 03.08.2022
(51) Int. Cl.: A61B 5/00, G16H 50/20, G16H 10/20, G06Q 30/06

(54) **SCALP TYPE DIAGNOSTIC SYSTEM ON BASIS OF SCALP STATE INFORMATION, AND SCALP IMPROVING METHOD USING SAME**

(30) Priority: 02.11.2021 KR 20210148436
(71) Applicant: Aramhuvis Co., Ltd., Gyeonggi-do 13605 (KR)
(72) Inventor: PARK, Dongsoon, Munkyeong-si Gyeongsangbuk-do 36923 (KR); JEONG, Jeongil, Seoul 08564 (KR)
(74) Representative: M. Zardi & Co S.A.
(86) International application number: PCT/KR2022/011489
(87) International publication number: WO 2023/080395

(57) **Abstract**

The present invention relates to a scalp type diagnostic system on the basis of scalp state information, and a scalp improving method using same, the system transmitting measurements of scalp questionnaire data and a scalp image to a server, storing same, and sharing the stored scalp questionnaire data and scalp image with a recommended service server and an artificial intelligence server to diagnose and analyze same, thereby accurately analyzing scalp state information with maximized speed and efficiency, diagnosing a scalp type on the basis of the analysis, carrying out a scalp improving method in accordance with the diagnosed scalp type, and recommending a suitable product in accordance with the scalp type.

## Description

### [Technical Field]

The present invention relates to a scalp type diagnostic system on the basis of scalp state information, and a scalp improving method using the same, and more specifically, to a scalp type diagnostic system on the basis of scalp state information, and a scalp improving method using the same the system, the system transmitting measurements of scalp questionnaire data and a scalp image to a server, storing the same, and sharing the stored scalp questionnaire data and scalp image with a recommended service server and an artificial intelligence (AI) server to diagnose and analyze the scalp questionnaire data and scalp image, thereby accurately analyzing scalp state information, diagnosing a scalp type on the basis of the analysis, carrying out a scalp improving method in accordance with the diagnosed scalp type, and recommending a suitable product in accordance with the scalp type.

In the present invention, the term "scalp state information" may be defined as ① micro keratin, ② excessive sebum, ③ erythema between hair follicles, ④ follicular erythema,⑤ pustules, ⑥ dandruff, and ⑦ hair loss (low hair density or small hair thickness), and
the term "scalp type" may be defined as 10 types: ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia.

### [Background Art]

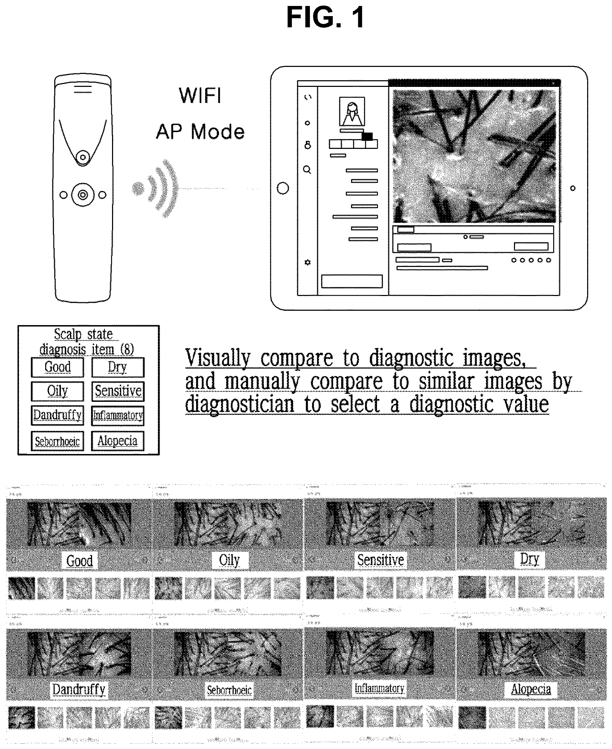

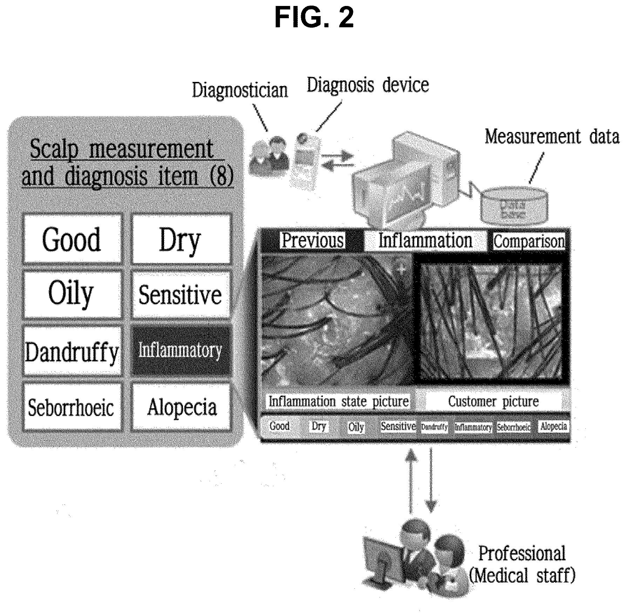

Conventionally, as shown in FIGS. 1 and 2, a diagnostician compares a reference image for each item with a diagnosis image with the naked eye to find a similar image and manually select a diagnosis value.

In other words, after a user measures a scalp as an image by using a diagnosis device, the diagnosis results for each diagnosis item (e.g., dry, sensitive, inflammatory, alopecia, good, oily, dandruffy, and seborrheic) needs to be identified later rather than in real time, and the diagnosis results (e.g., inflammatory among several items) may be obtained only when the analysis and diagnosis results for the scalp measurement data are input by an expert after visual comparison.

Such conventional technology has a problem in that a speed is slow until a diagnosis result is obtained and accuracy (less than 70%) is low.

In order to solve the above existing problems, the present applicant (inventor) has developed an "artificial intelligence scalp image diagnosis and analysis system using big data and a product recommendation system using the same" and has registered the same with the Korean Intellectual Property Office as Patent No. 10-2271558 (hereinafter, referred to as "the cited reference").

However, in the cited reference, an accurate diagnosis function through artificial intelligence (deep learning) image analysis may be implemented by an image of the scalp measured by a diagnostician, a diagnosis result may be confirmed in real time, a diagnosis result with high accuracy may be obtained, and a product suitable for a scalp state may be recommended according to the diagnosis result diagnosed by the artificial intelligence, but the scalp image measured by the diagnostician needs to pass through a main processor in order to proceed with an analysis by AI, and thus the effect and speed of the analysis are disadvantageous, the diagnosis of a scalp type based on scalp state information by the scalp diagnosis AI is not more various and specific, a diagnosis result according to the severity of the scalp state information is not presented, and a method for improving the scalp by the diagnosis result is not presented.

### [Disclosure]

### [Technical Problem]

Accordingly, the present invention has been made to solve the above-mentioned problems, and an object of the present invention is to improve Korean Patent No. 10-2271558 (titled an artificial intelligence scalp image diagnosis and analysis system using big data and a product recommendation system using the same), and to provide a scalp type diagnostic system on the basis of scalp state information and a scalp improving method using the same, the system transmitting measurements of scalp questionnaire data and a scalp image to a server, storing the same, and sharing the stored scalp questionnaire data and scalp image with a recommended service server and an artificial intelligence (AI) server to diagnose and analyze the scalp questionnaire data and scalp image, thereby accurately analyzing scalp state information, diagnosing a scalp type on the basis of the analysis, carrying out a scalp improving method in accordance with the diagnosed scalp type, and recommending a suitable product in accordance with the scalp type.

### [Technical Solution]

As a first embodiment to achieve the above object of the present invention, there may be provided:
a server for file storage, configured to transmit questionnaire information obtained by asking a subject as well as a scalp image obtained by one of a scalp diagnosis device or a terminal through API (RESTful) as a cloud service;
a main processor configured to diagnose the questionnaire information through a self algorithm among the questionnaire information directly received from the server for file storage or received through the API (RESTful) as the cloud service as well as the scalp images, analyze the scalp image for all or some of diagnosis items (for example, ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia) by artificial intelligence (AI) analysis of an artificial intelligence processor by utilizing information of big data constructed in database, and transmit diagnosis results from the analysis and diagnosis based on the questionnaire information in real time to a terminal of a diagnostician again through the API together with a recommended product in accordance with an appropriate prescription;
an artificial intelligence processor configured to perform AI analysis, which classifies the scalp image directly received from the server for file storage or received from the main processor into all or some of the diagnosis items (for example, some of ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia) by utilizing accumulated data of database;
a scalp diagnosis AI algorithm configured to receive information classified into all or some of the diagnostic items from the artificial intelligence processor and perform learning and reading by a deep learning algorithm to perform a specific precision diagnosis, thereby deriving a final result diagnosis; and
database configured to accumulate scalp measurement, diagnosis, and recommendation data to be provided to the main processor so as to perform self scalp diagnosis and recommendation service, in which
the scalp image may be classified into 10 scalp types based on seven pieces of scalp state information by utilizing the accumulated data of the database through the AI processor,
the seven pieces of "scalp state information" may include ① micro keratin, ② excessive sebum, ③ erythema between hair follicles, ④ follicular erythema, ⑤ pustules, ⑥ dandruff, and ⑦ hair loss (low hair density or small hair thickness), and
the 10 "scalp types" may include ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia.
The scalp image may be classified into the following 10 "scalp types" based on the seven pieces of "scalp state information" by an artificial intelligence processor:
   (1) good: micro keratin (X), excessive sebum (X), erythema between hair follicles (X), follicular erythema (X) or pustules (X), dandruff (X), hair loss (X);
   (2) dry: micro keratin (O), excessive sebum (X), erythema between hair follicles (X), follicular erythema (X) or pustules (X), dandruff (X), hair loss (X);
   (3) oily: micro keratin (X), excessive sebum (O), erythema between hair follicles (X), follicular erythema (X) or pustules (X), dandruff (X), hair loss (X);
   (4) sensitive: micro keratin (X), excessive sebum (X), erythema between hair follicles (O), follicular erythema (X) or pustules (X), dandruff (X), hair loss (X);
   (5) atopic: micro keratin (O), excessive sebum (X), erythema between hair follicles (O), follicular erythema (X) or pustules (X), dandruff (X), hair loss (X);
   (6) seborrheic: micro keratin (X), excessive sebum (O), erythema between hair follicles (O), follicular erythema (X) or pustules (X), dandruff (X), hair loss (X);
   (7) troublesome (inflammatory): micro keratin (X), excessive sebum (X), erythema between hair follicles (X), follicular erythema (O) or pustules (O), dandruff (X), hair loss (X);
   (8) dry dandruffy: micro keratin (O), excessive sebum (X), erythema between hair follicles (X), follicular erythema (X) or pustules (X), dandruff (O), hair loss (X);
   (9) oily dandruffy: micro keratin (X), excessive sebum (O), erythema between hair follicles (X), follicular erythema (X) or pustules (X), dandruff (O), hair loss (X); and
   (10) alopecia: micro keratin (X), excessive sebum (X), erythema between hair follicles (X), follicular erythema (X) or pustules (X), dandruff (X), hair loss (O), and
the scalp state information may be classified into ① micro keratin, ② excessive sebum, ③ erythema between hair follicles, ④ follicular erythema, ⑤ pustules, ⑥ dandruff, and ⑦ hair loss, may be divided into three stages of 0 (none), 1 (mild), 2 (moderate), and 3 (severe) depending on severity, and may be classified into a scalp type of a single symptom and all the scalp types of combination symptoms (① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia).

A sequence of improving the scalp according to the scalp type may be performed in an order of ① troublesome, ② seborrheic, ③ atopic, ④ oily dandruffy, ⑤ dry dandruffy, ⑥ sensitive, ⑦ alopecia, ⑧ oily, ⑨ dry, ⑩ good, and the sequence of improving may be sequentially performed for all or some of them.

As a second embodiment to achieve the above object of the present invention, there may be provided: an artificial intelligence (AI) processor configured to perform AI analysis, which receives a scalp image obtained from a subject by one of a scalp diagnosis device or a terminal through API (RESTful) as a cloud service, and classifies the received scalp image into all or some of diagnosis items (for example, some of ① good (normal), ② dry, ③ oily, ④ sensitive, ⑤ atopic, ⑥ seborrheic, ⑦ troublesome, ⑧ dry dandruffy, ⑨ oily dandruffy, ⑩ hair loss); and
a scalp diagnosis artificial intelligence (AI) algorithm configured to receive information classified into all or some of the diagnostic items from the artificial intelligence (AI) processor and perform learning and reading by a deep learning algorithm to perform a specific precision diagnosis, thereby deriving a final result diagnosis, in which
the scalp image is classified into 10 scalp types based on seven pieces of scalp state information by utilizing the accumulated data of the database through the artificial intelligence processor,
the seven pieces of "scalp state information" includes ① micro keratin, ② excessive sebum, ③ erythema between hair follicles, ④ follicular erythema, ⑤ pustules, ⑥ dandruff, and ⑦ hair loss (low hair density or small hair thickness), and
the 10 types of "scalp type" include ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia.

The scalp image may be classified into the following 10 types of "scalp type" based on the seven pieces of "scalp state information" by an artificial intelligence processor:
(1) good: micro keratin (X), excessive sebum (X), erythema between hair follicles (X), follicular erythema (X) or pustules (X), dandruff (X), hair loss (X);
(2) dry: micro keratin (O), excessive sebum (X), erythema between hair follicles (X), follicular erythema (X) or pustules (X), dandruff (X), hair loss (X);
(3) oily: micro keratin (X), excessive sebum (O), erythema between hair follicles (X), follicular erythema (X) or pustules (X), dandruff (X), hair loss (X);
(4) sensitive: micro keratin (X), excessive sebum (X), erythema between hair follicles (O), follicular erythema (X) or pustules (X), dandruff (X), hair loss (X);
(5) atopic: micro keratin (O), excessive sebum (X), erythema between hair follicles (O), follicular erythema (X) or pustules (X), dandruff (X), hair loss (X);
(6) seborrheic: micro keratin (X), excessive sebum (O), erythema between hair follicles (O), follicular erythema (X) or pustules (X), dandruff (X), hair loss (X);
(7) troublesome (inflammatory): micro keratin (X), excessive sebum (X), erythema between hair follicles (X), follicular erythema (O) or pustules (O), dandruff (X), hair loss (X);
(8) dry dandruffy: micro keratin (O), excessive sebum (X), erythema between hair follicles (X), follicular erythema (X) or pustules (X), dandruff (O), hair loss (X);
(9) oily dandruffy: micro keratin (X), excessive sebum (O), erythema between hair follicles (X), follicular erythema (X) or pustules (X), dandruff (O), hair loss (X); and
(10) alopecia: micro keratin (X), excessive sebum (X), erythema between hair follicles (X), follicular erythema (X) or pustules (X), dandruff (X), hair loss (O), and
the scalp state information may be classified into ① micro keratin, ② excessive sebum, ③ erythema between hair follicles, ④ follicular erythema, ⑤ pustules, ⑥ dandruff, and ⑦ hair loss, may be divided into three stages of 0 (none), 1 (mild), 2 (moderate), and 3 (severe) depending on severity, and may be classified into a scalp type of a single symptom and all the scalp types of combination symptoms (① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia).

A sequence of improving the scalp according to the scalp type may be performed in an order of ① troublesome, ② seborrheic, ③ atopic, ④ oily dandruffy, ⑤ dry dandruffy, ⑥ sensitive, ⑦ alopecia, ⑧ oily, ⑨ dry, ⑩ good, and the sequence of improving may be sequentially performed for all or some of them.

### [Advantageous Effects]

A "scalp type diagnostic system on the basis of scalp state information and a scalp improving method using the same" according to the present invention can transmit measurements of scalp questionnaire data and a scalp image to a server, store the same, and share the stored scalp questionnaire data and scalp image in a main processor and an artificial intelligence (AI) processor to diagnose and analyze the scalp questionnaire data and scalp image, thereby checking diagnosis results in real time, accurately analyzing scalp state information, diagnosing a scalp type on the basis of the analysis, carrying out a scalp improving method in accordance with the diagnosed scalp type, and recommending a suitable improving method and a suitable product in accordance with the scalp type.

In addition, the scalp image input in the server for file storage can be shared in the main processor and the artificial intelligence (AI), thereby providing an effect of reducing a load of having to upload separate scalp images to each server once and achieving an advantageous speed.

### [Description of Drawings]

FIGS. 1 and 2 are views showing a method for manually diagnosing scalp with the naked eye according to the related art.
FIGS. 3 and 4 are overall block diagrams showing a scalp diagnosis function through questionnaire data analysis and artificial intelligence (deep learning) image analysis as a "scalp type diagnostic system on the basis of scalp state information and a scalp improving method using the same" according to the present invention.
FIG. 5 is a scalp type algorithm by classification of scalp symptoms according to the present invention.

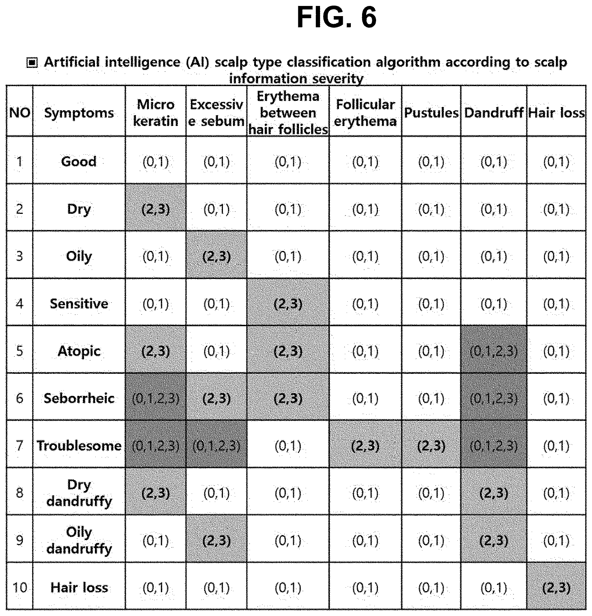

FIG. 6 is an artificial intelligence (AI) scalp type classification algorithm according to the severity of scalp information according to the present invention.
FIG. 7 is a flowchart of scalp improvement according to the present invention.
FIG. 8 is a structural diagram of an EfficientNet model as a deep learning algorithm in a "scalp type diagnostic system on the basis of scalp state information and a scalp improving method using the same" according to the present invention.
FIG. 9 is a basic block depthwise convolution structural diagram of EfficientNet as a deep learning algorithm in a "scalp type diagnostic system on the basis of scalp state information and a scalp improving method using the same" according to the present invention.
FIG. 10 is a basic block depthwise convolution structural diagram of EfficientNet as a deep learning algorithm in a "scalp type diagnostic system on the basis of scalp state information and a scalp improving method using the same" according to the present invention.

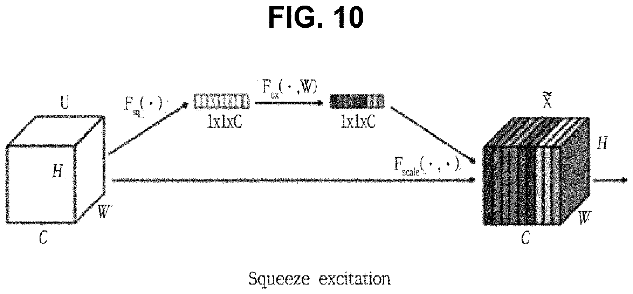

FIG. 10 is a basic block squeeze excitation structural diagram of EfficientNet as a deep learning algorithm in a "scalp type diagnostic system on the basis of scalp state information and a scalp improving method using the same" according to the present invention.

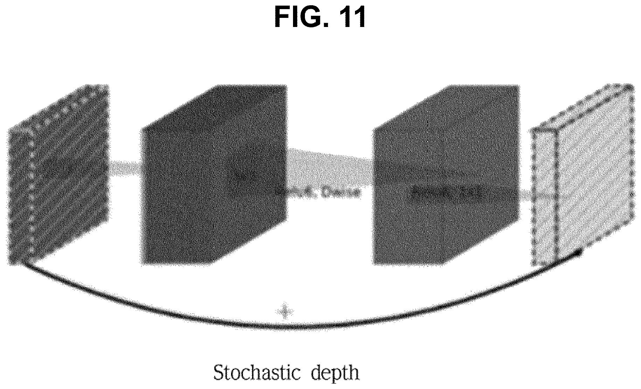

FIG. 11 is a basic block stochastic depth structural diagram of EfficientNet as a deep learning algorithm in a "scalp type diagnostic system on the basis of scalp state information and a scalp improving method using the same" according to the present invention.

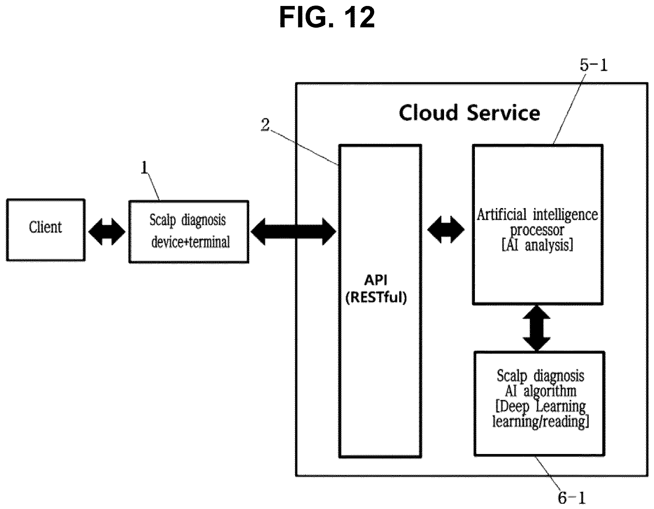

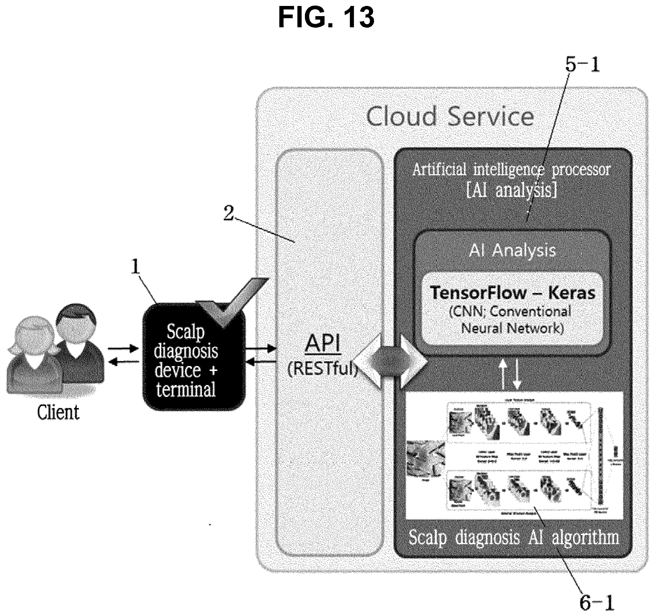

FIGS. 12 and 13 are overall block diagrams showing a scalp diagnosis function through artificial intelligence (deep learning) image analysis as another "scalp type diagnostic system on the basis of scalp state information and a scalp improving method using the same" according to the present invention.

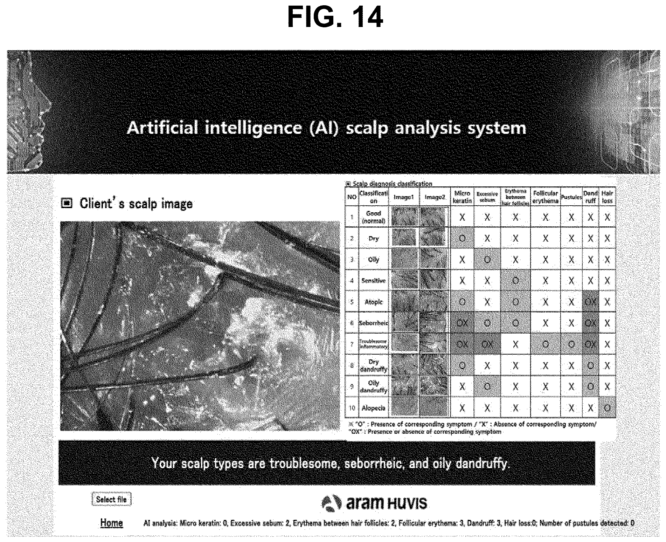

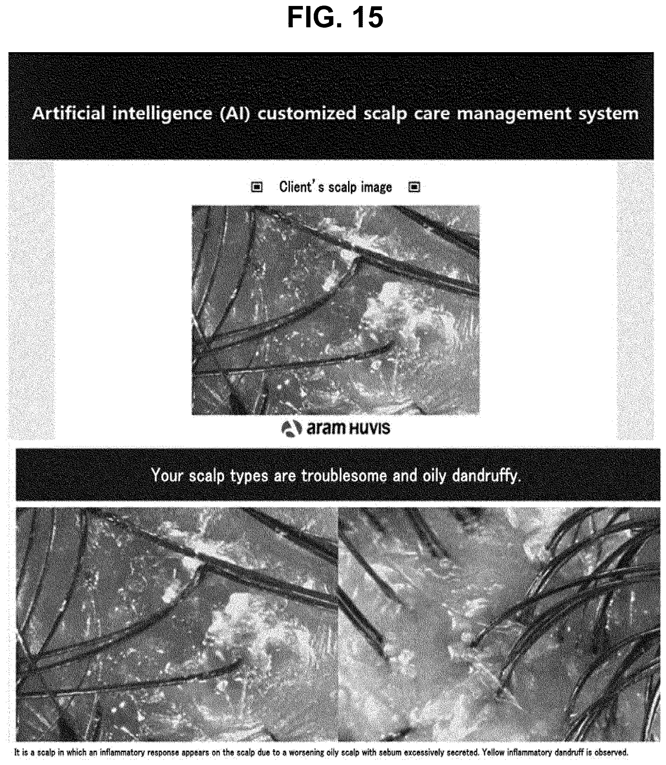

FIGS. 14 and 15 are exemplary views showing that a diagnostician's scalp diagnosis may be accurately performed and real-time diagnosis results may be rapidly obtained through the scalp questionnaire and scalp image analysis in a "scalp type diagnostic system on the basis of scalp state information and a scalp improving method using the same" according to the present invention.

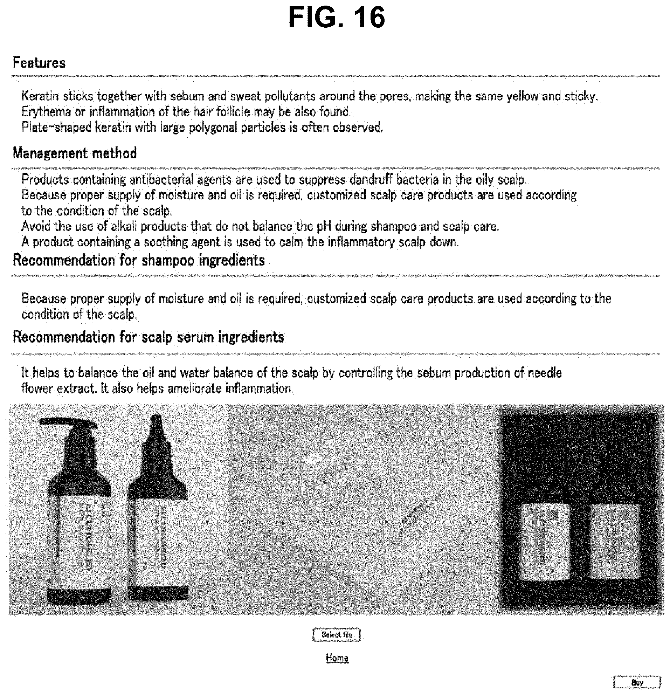

FIG. 16 is an exemplary view showing that a sequence of scalp improvement, a prescription and a customized product by a diagnostician (for a user) are recommended according to final analysis results in a "scalp type diagnostic system on the basis of scalp state information and a scalp improving method using the same" according to the present invention.

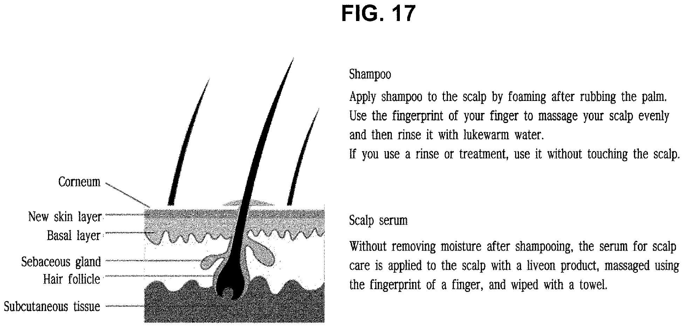

FIG. 17 is an exemplary view showing that product recommendation based on the results of scalp diagnosis is defined as shampoo and scalp serum and showing product segmentation and algorithm in a "scalp type diagnostic system on the basis of scalp state information and a scalp improving method using the same" according to the present invention.

### Best Mode

Hereinafter, preferred embodiments will be described in detail according to the accompanying drawings.

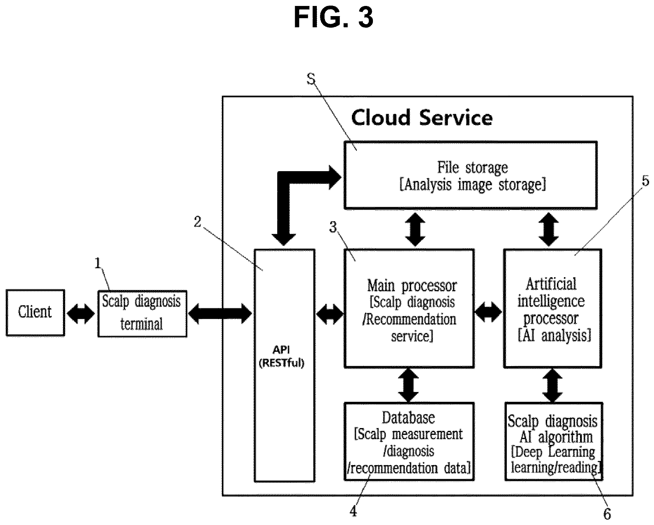

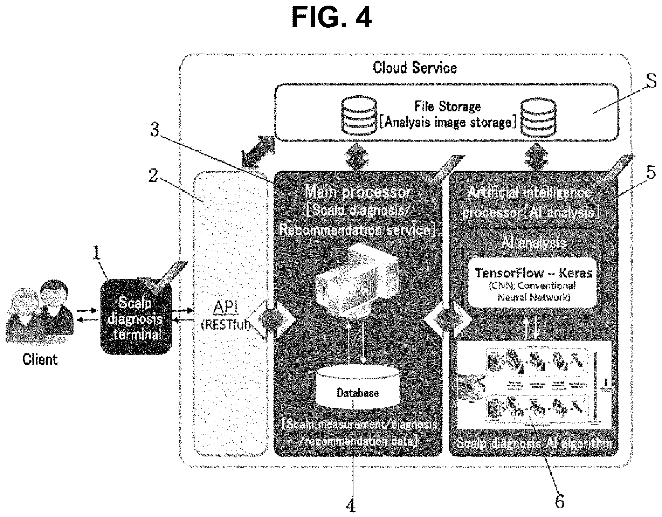

First, embodiments will be described as a scalp type diagnostic system on the basis of scalp state information and a scalp improving method using the same" of the present invention according to FIGS. 3 and 4 and will be described with reference to the accompanying drawings according to the need for explanation.

As a first embodiment of the present invention, there will be provided a server S for file storage configured to transmit questionnaire information obtained through a questionnaire answered by a subject and a scalp image obtained by one of a scalp diagnosis device or a terminal 1 through API (RESTful) as a cloud service.

There may be provided a main processor 3 configured to diagnose the questionnaire information through a self algorithm among the questionnaire information directly received from the server S for file storage or received through the API (RESTful) 2 as the cloud service as well as the scalp images, analyze the scalp image for all or some of diagnosis items (for example, ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia) by artificial intelligence (AI) analysis of an artificial intelligence processor by utilizing information of big data constructed in database 4, and transmit diagnosis results from the analysis and diagnosis based on the questionnaire information in real time to a terminal of a diagnostician again through the API together with a recommended product in accordance with an appropriate prescription.

There may be provided an artificial intelligence processor 5 configured to perform AI analysis, which classifies the scalp image directly received from the server S for file storage or received from the main processor 3 into all or some of the diagnosis items (for example, some of ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia) by utilizing accumulated data of database 4.

There may be provided a scalp diagnosis artificial intelligence (AI) algorithm 6 configured to receive information classified into all or some of the diagnostic items from the AI processor 5 and perform learning and reading by a deep learning algorithm to perform a specific precision diagnosis, thereby deriving a final result diagnosis, and database 4 configured to accumulate scalp measurement, diagnosis, and recommendation data to be provided to the main processor so as to perform self scalp diagnosis and recommendation service.

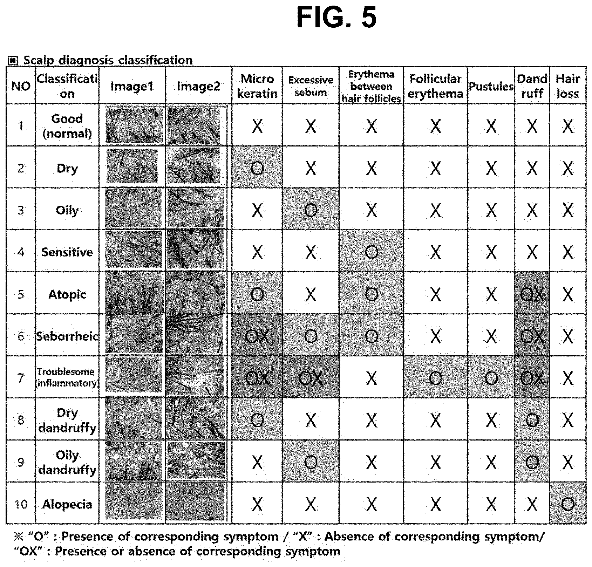

The scalp image may be classified into 10 scalp types based on seven pieces of scalp state information by utilizing the accumulated data of the database through the artificial intelligence processor 5 (see FIG. 5),
the seven pieces of "scalp state information" may include ① micro keratin, ② excessive sebum, ③ erythema between hair follicles, ④ follicular erythema, ⑤ pustules, ⑥ dandruff, and ⑦ hair loss (low hair density or small hair thickness), and
the 10 "scalp types" may include ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia.

The scalp image may be classified into the following 10 "scalp types" based on the seven pieces of "scalp state information" by an artificial intelligence processor (see FIG. 5):
(1) good: micro keratin (X), excessive sebum (X), erythema between hair follicles (X), follicular erythema (X) or pustules (X), dandruff (X), hair loss (X);
(2) dry: micro keratin (O), excessive sebum (X), erythema between hair follicles (X), follicular erythema (X) or pustules (X), dandruff (X), hair loss (X);
(3) oily: micro keratin (X), excessive sebum (O), erythema between hair follicles (X), follicular erythema (X) or pustules (X), dandruff (X), hair loss (X);
(4) sensitive: micro keratin (X), excessive sebum (X), erythema between hair follicles (O), follicular erythema (X) or pustules (X), dandruff (X), hair loss (X);
(5) atopic: micro keratin (O), excessive sebum (X), erythema between hair follicles (O), follicular erythema (X) or pustules (X), dandruff (X), hair loss (X);
(6) seborrheic: micro keratin (X), excessive sebum (O), erythema between hair follicles (O), follicular erythema (X) or pustules (X), dandruff (X), hair loss (X);
(7) troublesome (inflammatory): micro keratin (X), excessive sebum (X), erythema between hair follicles (X), follicular erythema (O) or pustules (O), dandruff (X), hair loss (X);
(8) dry dandruffy: micro keratin (O), excessive sebum (X), erythema between hair follicles (X), follicular erythema (X) or pustules (X), dandruff (O), hair loss (X);
(9) oily dandruffy: micro keratin (X), excessive sebum (O), erythema between hair follicles (X), follicular erythema (X) or pustules (X), dandruff (O), hair loss (X); and
(10) alopecia: micro keratin (X), excessive sebum (X), erythema between hair follicles (X), follicular erythema (X) or pustules (X), dandruff (X), hair loss (O), and
the scalp state information may be classified into ① micro keratin, ② excessive sebum, ③ erythema between hair follicles, ④ follicular erythema, ⑤ pustules, ⑥ dandruff, and ⑦ hair loss, may be divided into three stages of 0 (none), 1 (mild), 2 (moderate), and 3 (severe) depending on severity, and may be classified into a scalp type of a single symptom and all the scalp types of combination symptoms (① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia).

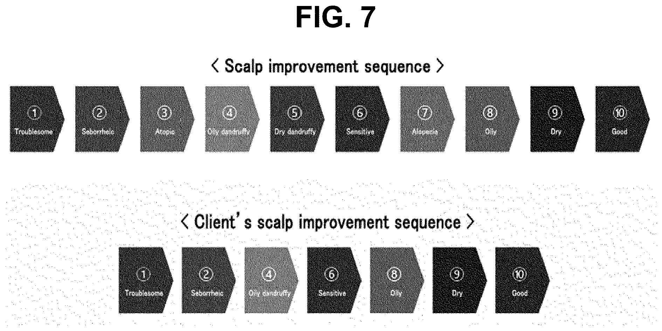

A sequence of improving the scalp according to the scalp type may be performed in an order of ① troublesome, ② seborrheic, ③ atopic, ④ oily dandruffy, ⑤ dry dandruffy, ⑥ sensitive, ⑦ alopecia, ⑧ oily, ⑨ dry, ⑩ good, and the sequence of improving may be sequentially performed for all or some of them (see FIG. 7).

Then, as a second embodiment of the present invention, as shown in FIGS. 12 and 13, there may be provided an artificial intelligence processor 5-1 configured to perform AI analysis, which receives a scalp image obtained from a subject by one of a scalp diagnosis device or a terminal 1 through API (RESTful) 2 as a cloud service, and classifies the received scalp image into all or some of diagnosis items (for example, some of ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia).

There may be provided a scalp diagnosis artificial intelligence (AI) algorithm 6-1 configured to receive information classified into all or some of the diagnostic items from the AI processor 5-1 and perform learning and reading by a deep learning algorithm to perform a specific precision diagnosis, thereby deriving a final result diagnosis.

The scalp image may be classified into 10 scalp types based on seven pieces of scalp state information by utilizing the accumulated data of the database through the artificial intelligence processor 5-1.

The seven pieces of "scalp state information" may include ① micro keratin, ② excessive sebum, ③ erythema between hair follicles, ④ follicular erythema, ⑤ pustules, ⑥ dandruff, and ⑦ hair loss (low hair density or small hair thickness), and
the 10 "scalp types" may include ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia.

The scalp image may be classified into the following 10 "scalp types" based on the seven pieces of "scalp state information" by an artificial intelligence processor:
(1) good: micro keratin (X), excessive sebum (X), erythema between hair follicles (X), follicular erythema (X) or pustules (X), dandruff (X), hair loss (X);
(2) dry: micro keratin (O), excessive sebum (X), erythema between hair follicles (X), follicular erythema (X) or pustules (X), dandruff (X), hair loss (X);
(3) oily: micro keratin (X), excessive sebum (O), erythema between hair follicles (X), follicular erythema (X) or pustules (X), dandruff (X), hair loss (X);
(4) sensitive: micro keratin (X), excessive sebum (X), erythema between hair follicles (O), follicular erythema (X) or pustules (X), dandruff (X), hair loss (X);
(5) atopic: micro keratin (O), excessive sebum (X), erythema between hair follicles (O), follicular erythema (X) or pustules (X), dandruff (X), hair loss (X);
(6) seborrheic: micro keratin (X), excessive sebum (O), erythema between hair follicles (O), follicular erythema (X) or pustules (X), dandruff (X), hair loss (X);
(7) troublesome (inflammatory): micro keratin (X), excessive sebum (X), erythema between hair follicles (X), follicular erythema (O) or pustules (O), dandruff (X), hair loss (X);
(8) dry dandruffy: micro keratin (O), excessive sebum (X), erythema between hair follicles (X), follicular erythema (X) or pustules (X), dandruff (O), hair loss (X);
(9) oily dandruffy: micro keratin (X), excessive sebum (O), erythema between hair follicles (X), follicular erythema (X) or pustules (X), dandruff (O), hair loss (X); and
(10) alopecia: micro keratin (X), excessive sebum (X), erythema between hair follicles (X), follicular erythema (X) or pustules (X), dandruff (X), hair loss (O), and
the scalp state information may be classified into ① micro keratin, ② excessive sebum, ③ erythema between hair follicles, ④ follicular erythema, ⑤ pustules, ⑥ dandruff, and ⑦ hair loss, may be divided into three stages of 0 (none), 1 (mild), 2 (moderate), and 3 (severe) depending on severity, and may be classified into a scalp type of a single symptom and all the scalp types of combination symptoms (① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia).

A sequence of improving the scalp according to the scalp type may be performed in an order of ① troublesome, ② seborrheic, ③ atopic, ④ oily dandruffy, ⑤ dry dandruffy, ⑥ sensitive, ⑦ alopecia, ⑧ oily, ⑨ dry, ⑩ good, and the sequence of improving may be sequentially performed for all or some of them.

### [Mode for Invention]

Hereinafter, preferred embodiments will be described in detail according to the accompanying drawings.

Terms defined in describing the present invention are defined in consideration of functions, forms, or the like in the present invention, and should not be understood as limiting the technical components of the present invention.

The present invention may be variously modified and take on various forms, and thus embodiments (aspects) (or examples) will be described herein in more detail. However, this is not intended to limit the present invention to a certain disclosure form, but shall be construed to include all the modifications, equivalents or substitutes within the spirit and technical scope of the present invention.

In addition, in each drawing, the size or thickness of components may be exaggerated or expressed in a large (or thick), small (or thin) or simplified form in consideration of convenience of understanding, etc., but the scope of the present invention should not be construed as being limited thereto.

Terms used in the present application are used only to describe a certain embodiment (aspect) (or example) and are not intended to limit the present invention.

Such terms as those defined in a generally used dictionary are to be interpreted to have the meanings equal to the contextual meanings in the relevant art, and are not to be interpreted to have ideal or excessively formal meanings, unless clearly defined in the present application.

Embodiments for achieving an object of the present invention will be described with reference to accompanying drawings.

First, a description will be provided according to FIGS. 3 to 4.

As a first embodiment to achieve the above object of the present invention, there will be provided a server S for file storage configured to transmit questionnaire information obtained through a questionnaire answered by a subject and a scalp image obtained by one of a scalp diagnosis device or a terminal 1 through API (RESTful) 2 as a cloud service.

There may be provided a main processor 3 configured to diagnose the questionnaire information through a self algorithm among the questionnaire information directly received from the server S for file storage or received through the API (RESTful) 2 as the cloud service as well as the scalp images, analyze the scalp image for all or some of diagnosis items (for example, ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia) by artificial intelligence (AI) analysis of an artificial intelligence processor by utilizing information of big data constructed in database 4, and transmit diagnosis results from the analysis and diagnosis based on the questionnaire information in real time to a terminal of a diagnostician again through the API together with a recommended product in accordance with an appropriate prescription.

There may be provided an artificial intelligence processor 5 configured to perform AI analysis, which classifies the scalp image directly received from the server S for file storage or received from the main processor 3 into all or some of the diagnosis items (for example, some of ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia) by utilizing accumulated data of database 4.

There may be provided a scalp diagnosis artificial intelligence algorithm 6 configured to receive information classified into all or some of the diagnostic items from the AI processor 5 and perform learning and reading by a deep learning algorithm to perform a specific precision diagnosis, thereby deriving a final result diagnosis, and database 4 configured to accumulate scalp measurement, diagnosis, and recommendation data to be provided to the main processor so as to perform self scalp diagnosis and recommendation service.

The scalp image may be classified into 10 scalp types based on seven pieces of scalp state information by utilizing the accumulated data of the database through the artificial intelligence processor 5 (see FIG. 5),
the seven pieces of "scalp state information" may include ① micro keratin, ② excessive sebum, ③ erythema between hair follicles, ④ follicular erythema, ⑤ pustules, ⑥ dandruff, and ⑦ hair loss (low hair density or small hair thickness), and
the 10 "scalp types" may include ① good (normal), ② dry, ③ oily, ④ sensitive, ⑤ atopic, ⑧ seborrheic, ⑦ troublesome, ⑧ dry dandruffy, ⑨ oily dandruffy, and ⑩ alopecia.

The scalp image may be classified into the following 10 "scalp types" based on the seven pieces of "scalp state information" by an artificial intelligence processor (see FIG. 5):
(1) good: micro keratin (X), excessive sebum (X), erythema between hair follicles (X), follicular erythema (X) or pustules (X), dandruff (X), hair loss (X);
(2) dry: micro keratin (O), excessive sebum (X), erythema between hair follicles (X), follicular erythema (X) or pustules (X), dandruff (X), hair loss (X);
(3) oily: micro keratin (X), excessive sebum (O), erythema between hair follicles (X), follicular erythema (X) or pustules (X), dandruff (X), hair loss (X);
(4) sensitive: micro keratin (X), excessive sebum (X), erythema between hair follicles (O), follicular erythema (X) or pustules (X), dandruff (X), hair loss (X);
(5) atopic: micro keratin (O), excessive sebum (X), erythema between hair follicles (O), follicular erythema (X) or pustules (X), dandruff (X), hair loss (X);
(6) seborrheic: micro keratin (X), excessive sebum (O), erythema between hair follicles (O), follicular erythema (X) or pustules (X), dandruff (X), hair loss (X);
(7) troublesome (inflammatory): micro keratin (X), excessive sebum (X), erythema between hair follicles (X), follicular erythema (O) or pustules (O), dandruff (X), hair loss (X);
(8) dry dandruffy: micro keratin (O), excessive sebum (X), erythema between hair follicles (X), follicular erythema (X) or pustules (X), dandruff (O), hair loss (X);
(9) oily dandruffy: micro keratin (X), excessive sebum (O), erythema between hair follicles (X), follicular erythema (X) or pustules (X), dandruff (O), hair loss (X); and
(10) alopecia: micro keratin (X), excessive sebum (X), erythema between hair follicles (X), follicular erythema (X) or pustules (X), dandruff (X), hair loss (O), and
the scalp state information may be classified into ① micro keratin, ② excessive sebum, ③ erythema between hair follicles, ④ follicular erythema, ⑤ pustules, ⑥ dandruff, and ⑦ hair loss, may be divided into three stages of 0 (none), 1 (mild), 2 (moderate), and 3 (severe) depending on severity, and may be classified into a scalp type of a single symptom and all the scalp types of combination symptoms (① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia).

A sequence of improving the scalp according to the scalp type may be performed in an order of ① troublesome, ② seborrheic, ③ atopic, ④ oily dandruffy, ⑤ dry dandruffy, ⑥ sensitive, ⑦ alopecia, ⑧ oily, ⑨ dry, ⑩ good, and the sequence of improving may be sequentially performed for all or some of them (see FIG. 7).

The features and step classification of the scalp state information may be as follows.

### 1. Micro keratin

### 1) Features

- Lack of natural moisturizing factors (NMF) on a scalp surface and a decline in sebaceous gland functions, resulting in early bullosa symptoms.
- Fine keratin observed and annual ring-shaped keratin layers formed around pores.
- Complaint of skin tightening or itching due to lack of oil and moisture.

### 2) Step classification (classification by severity);

* Step 3: ①. Neither erythema nor excessive sebum with a large amount of small and powdery keratin.

②. Annual ring-shaped surface around scalp pores.

* Step 2: ①. Neither erythema nor excessive sebum with occasional presence of small and powdery keratin.

②. Cracks on the scalp.

* Step 1: ①. Neither erythema nor excessive sebum with small and powdery keratin.

### 2. Excessive sebum

### 1) Features

- Glossy scalp surface due to excessive sebum secretion, and turbid scalp color.
- Seemingly stagnant water in pores and low hair density.
- In severe cases, accompanying odor, greasy and dandruffy hair, and seborrheic inflammation caused by sebum oxidation and bacteria.

### 2) Step classification (classification by severity);

* Step 3: ①. Excessive erythema or sebum, resulting in light reflection and glittering on the scalp and hair.

②. Stagnant sebum in pores.

* Step 2: ①. Excessive erythema or sebum, resulting in light reflection on the scalp and hair.

②. Slight sebum in pores.

* Step 1: ①. Excessive erythema or sebum, resulting in light reflection on the scalp and hair.

### 3. Erythema between hair follicles

### 1) Features

- Erythema or hemorrhage due to telangiectasia in the scalp.
- Vulnerable to redness and pain in the scalp even with mild stimuli such as solar heat or air-conditioned breeze.

### 2) Step classification (classification by severity);

* Step 3: ①. Very severe red erythema on the whole.

②. Capillary observed on the scalp.

* Step 2: ①. Clear erythema observed throughout the scalp.

* Step 1: ①. Pale pink erythema partially observed in the scalp.

### 4. Follicular erythema/pustules

### 1) Features

- Redness in the scalp with erythema on a surface thereof, in some cases.
- Inflammation caused by bacterial infection.

### 2) Step classification (classification by severity);

* Step 3: ①. Erythema in the scalp and clearly visible pustules around hair follicles.

* Step 2: ①. Erythema in the scalp and excessive sebum or visible keratin.

* Step 1: ①. Erythema in the scalp and scalp swelling sometimes observed.

### 5. Dandruff

### 1) Features

- Keratin produced and eliminated compared to normal scalp due to abnormal keratinization cycle.
- Malassezia yeast as a main cause.
- Dandruffy scalp affected by hormone, nutritional status, shampoo habit, stress, diet, etc.

### 2) Step classification (classification by severity);

* Step 3: ①. White keratin stacked in layers.

②. Keratin found in at least 60% of diagnosis area.

* Step 2: ①. Keratin area accounting for 30 to 60% of the scalp area upon diagnosis.

* Step 1: ①. Severe keratin and white portions of keratin piled up here and there.

②. Keratin found in 30% or less of diagnosis area.

### 6. Hair loss (low hair density or small hair thickness)

### 1) Features

- Absence of hair in an area where hair should be located or a rapid progress of vellus hair.
- Caused by a combination of inheritance, hormone and environment.
- Said to be accompanied by itching and increase in dandruff and sebum secretion in the case of environmental hair loss.
- No particular abnormality observed and rapid decrease in density in the case of genetic hair loss.

### 2) Step classification (classification by severity);

* Step 3: ①. Low density and thin thickness of hair on the whole.

②. At least two empty pores observed upon scalp diagnosis.

* Step 2: ①. Low density and thin thickness of hair.

②. Two connected hairs in one pore in many cases upon scalp diagnosis.

* Step 1: ①. Low density of hair, but partially thick hair.

### 7. Normal

### 1) Features

- Uniform and clear blue-white color on a scalp surface.
- Constant density of hair and free of keratin or impurities.
- Open pore and at least one strand of hair observed in one pore with similar hair thickness.
- Neither greasy nor dry hair observed even without shampooing for about a day upon questionnaire.

### 2) Step classification (classification by severity);

* Step 0 (Zero): Scalp without erythema, excessive sebum secretion, and keratin.

### * Features of the scalp type, and improvement method and sequence;

1. Problematic scalp may be reclassified into troublesome scalp, seborrheic scalp, and atopic scalp, which are inflammatory scalp.

Scalp inflammation may be accompanied by itching, and lead to metastasis in other areas when touched or scratched.

Thus, scalp inflammation needs to be preferentially ameliorated in scalp management.

Inflammation may be generally classified into troublesome scalp accompanied by inflammation, such as hair follicle erythema or follicle pustule (folliculitis), due to excessive proliferation of microorganisms (bacteria or fungi) on a scalp surface or a change in a scalp environment, seborrheic scalp which may cause a dandruff form on the erythema between hair follicles with excessive sebum secretion, and may be accompanied by inflammation, and atopic scalp with scalp epidermal proliferation and inflammation as representative symptoms caused by abnormal proliferation of Staphylococcus aureus on the scalp. Among them, a top priority may be given to ameliorating the troublesome scalp, so that scalp microorganisms may be managed through antibacterial and antifungal management. After that, seborrheic scalp and atopic scalp, which are the chronic inflammatory scalp, may be managed.

For the sequence of ameliorating the seborrheic scalp and the atopic scalp, it may be necessary to ameliorate the seborrheic scalp first, which has a blocked pore state, is sensitive to the scalp due to bacterial inflammation and is likely to be accompanied by hair loss. The atopic scalp may properly require antibacterial and moisturizing management for Staphylococcus aureus, and thus may be in a third place in the sequence of improvement.

2. Then, it may be necessary to manage oily dandruff and dry dandruff, which are not accompanied by inflammation and require antimicrobial management.

Dandruffy scalp may be caused by the proliferation of dandruff bacteria and may be accompanied by itching.

Dandruff may be classified into dry dandruff and oily dandruff, and the management of dry dandruff and intellectual dandruff may need to be different.

Oily dandruffy scalp may be a scalp symptom caused by a mixture of excessively secreted sebum and keratin, and may be accompanied by itching and odor. If the oily dandruffy scalp is left unattended, excessive sebum may cause inflammation and worsen symptoms, and thus oily dandruff may be managed first dry dandruff may be managed later.

Thus, the oily dandruffy scalp may be in a fourth place, and the dry dandruffy scalp may be in a fifth place.

3. The sensitive scalp may show thin capillaries in the scalp, may turn red when scratched, and may be accompanied by itching and irritation. The sensitive scalp may not immediately show a symptom, but may be accompanied by symptoms such as bacterial infection and inflammation due to external factors, and thus the sensitive scalp may be classified as problematic scalp, and may be in a sixth place in an improvement sequence.

4. In addition to inflammatory or bacterial factors, the alopecia scalp, which does not show any abnormalities in the scalp, but does not maintain a certain number of hair and shows a decrease in the number of hair, may be in a seventh place.

The alopecia scalp may be a scalp which needs to be managed for a long time, and the scalp which may progress to hair loss due to inflammation or bacterial may be first managed, and then a product may be mixed with an effective substance which may help the growth of hair later on.

5. Then, in addition to the problematic scalp, there may be a sequence for ameliorating the oily scalp, the dry scalp, and the normal scalp, which are classified according to a degree of sebum in the scalp.

The oily scalp may be naturally rich in oil and thus may correspond to sticky and oily scalp.

Due to excessive oil, fine dust or contaminants may be prone to sticking to the oily scalp, and oxidized sebum or adsorbed contaminants may cause a secondary problem. Thus, the oily scalp may need to be managed before the dry scalp.

For this reason, a sequence for ameliorating the oily scalp may be in an eighth place, and the dry scalp with insufficient scalp oil may be in a ninth place, and a good scalp in the best condition may be in a tenth place.

Meanwhile, the main processor 3 may be configured to diagnose the questionnaire information through a self algorithm constructed in the main processor 3 among the questionnaire information received from a subject as well as the scalp image. In other words, the main processor may receive scalp information data constructed from database and may diagnose the questionnaire information received from a diagnostician by a self algorithm.

In addition, the main processor may be configured to analyze the scalp state information and severity through artificial intelligence (AI) analysis by utilizing information of big data constructed in database, thereby analyzing into seven categories of ① micro keratin, ② excessive sebum, ③ erythema between hair follicles, ④ follicular erythema, ⑤ pustules, ⑥ dandruff, and ⑦ hair loss, analyze resulting scalp types into some of ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia, and transmit diagnosis results from the analysis and diagnosis based on the questionnaire information in real time to a terminal of a diagnostician again through the API together with a recommended product in accordance with an appropriate prescription.

The artificial intelligence processor 5 may learn the scalp and collect data as a deep learning step for scalp classification by artificial intelligence (AI) analysis by utilizing information of big data on the received scalp image, may perform labeling for the collected learning data, may perform classification learning and verification of the collected data with learning data and test data (8:2), and may derive an inference model (convolutional neural network: CNN).

For example, as a method for developing an inference model, TensorFlow, which is a deep learning library as a way for development using PyThon, may be used to classify the scalp (CNN: object recognition) through EfficientNet model re-training, and an inference model may be created through transfer learning with the EfficientNet model to make a comparison and make an interference with optimal accuracy, and thus the scalp state information and severity may be analyzed as a whole or in part (for example, ① micro keratin, ② excessive sebum, ③ erythema between hair follicles, ④ follicular erythema, ⑤ pustules, ⑥ dandruff, and ⑦ hair loss).

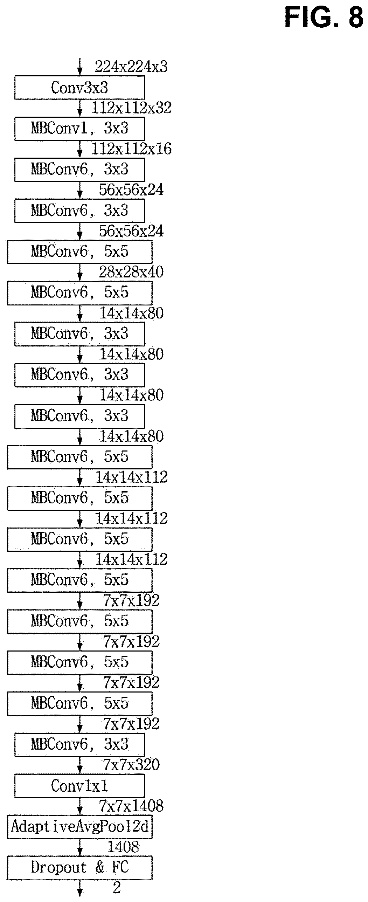

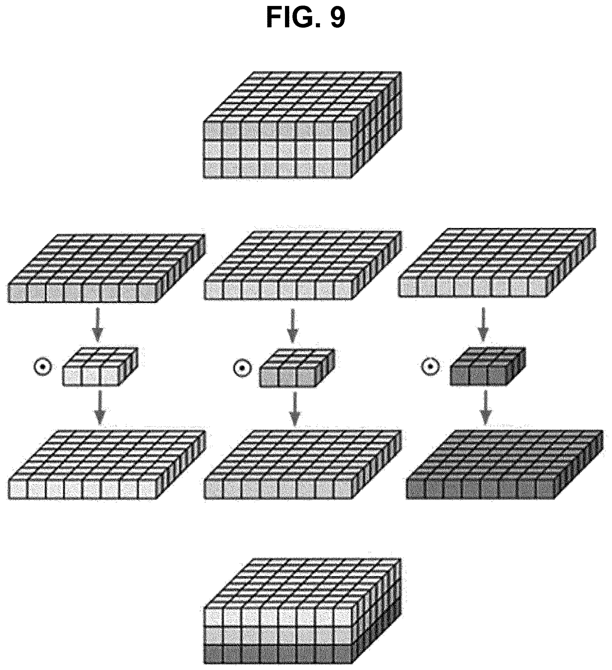

The EfficientNet model (see FIGS. 8 to 11) may include MBConv as a basic block of the EfficientNet.
- The MBConv may include general Conv layers for performing Depthwise Conv*, Squeeze excitation*, and width scaling up.
- Based on an EfficientNet b0 model, a total number of Conv blocks may be 18, two blocks at both ends may be Conv, and the rest may be MBConv.
- The entire Conv block may be divided into nine parts by combining the repeated blocks. Stochastic depth* may be applied only to the first MBConv of ③④⑤⑦ after width scaling up.

* Depthwise convolution: One of the grouped convolutions as a convolution technique which combines results by applying different 2D filters for each channel. A number of parameters may be reduced, and thus there may be an advantage of reducing an amount of computation and increasing a speed.

* Stochastic depth: Dropout of a channel unit.

* Squeeze excitation: After one value per channel is extracted with an AdaptiveAvgPool2d, the extracted value may be passed through a conv filter to reduce a number of channels and restore a number of channels. Then, an input image may be multiplied by a value passed through a sigmoid. This may be equal to considering the importance for each channel.

The scalp diagnosis AI algorithm 6 may be configured to receive a diagnosed scalp image from the artificial intelligence processor 5, perform learning and reading for the received classification information by using the EfficientNet model (see FIG. 8) as a deep learning algorithm utilizing information of big data of the database, infer an image through an additional retraining of a scalp image set to analyze the scalp state information and resulting severity into seven categories (① micro keratin, ② excessive sebum, ③ erythema between hair follicles, ④ follicular erythema, ⑤ pustules, ⑥ dandruff, and ⑦ hair loss), perform a precision diagnosis on the resulting scalp type for all or some of items: ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia by the scalp diagnosis AI algorithm to derive a final result diagnosis.

The diagnosis method may be a method using an EfficientNet deep learning model, but is not limited thereto, and a Google deep learning model or other deep learning models may be also used.

The derived final result diagnosis may be transmitted to the main processor 3 through the artificial intelligence processor 5.

It may provide scalp measurement, diagnosis, and recommendation data to the main processor so that the data may be accumulated, learned and read.

After that, it may transmit scalp image diagnosis results and diagnosis based on the questionnaire information in real time again to a terminal of a diagnostician again through the API together with a recommended product in accordance with an appropriate prescription.

As shown in FIGS. 14 and 15, the present invention may give precision to a diagnostician's scalp diagnosis and rapidly obtain real-time diagnosis results through scalp questionnaire and scalp image analysis.

In other words, when the diagnostician (user) inputs the scalp questionnaire information and the scalp image through the terminal, the final analysis results may be immediately obtained in real time by AI analysis as described in detail above.

When the final analysis result of the scalp according to the AI analysis is obtained, a sequence for improving skin, a prescription and a customized product of the diagnostician (user) according to the final analysis result may be recommended as shown in FIG. 16.

In other words, the customized product of the diagnostician (user) may be recommended by various algorithms according to the final analysis result of the scalp.

For example, product recommendation based on a scalp diagnosis result may be defined as shampoo and scalp serum, and product segmentation and algorithm are as shown in FIG. 17.

**[Table 1]**

| | | | | |
|---|---|---|---|---|
| Scalp type ① | Scalp type ② | Scalp type ③ | Scalp type ④ | Scalp type ⑤ |
| Good | Dry | Oily | Sensitive | Atopic |
| Scalp type ⑥ | Scalp type ⑦ | Scalp type ⑧ | Scalp type ⑨ | Scalp type ⑩ |
| Seborrheic | Troublesome | Dry dandruffy | Oily dandruffy | Hair loss |

* Classification of artificial intelligence (AI) scalp diagnosis scalp type based on scalp state information for product recommendation through artificial intelligence analysis/diagnosis of user's scalp measurement image

**[Table 2]**

| **No** | **Classifi cation** | **Scalp image AI analysis** | | | | | | **Scalp type determination** | |
|---|---|---|---|---|---|---|---|---|---|
| | | **Micro keratin** | **Excessive sebum** | **Erythema between hair follicles** | **Follicula r erythem a/pustule s** | **Dandr uffy** | **Hair loss** | **Scalp type** | **Detailed scalp type** |
| 1 | Good | (0,1) | (0,1) | (0,1) | (0,1) | (0,1) | (0,1) | A-Good | A-1 Good |
| 2 | Dry | 2 | (0,1) | (0,1) | (0,1) | (0,1) | (0,1) | B-Dry | B-1 Dry mild |
| | | 3 | (0,1) | (0,1) | (0,1) | (0,1) | (0,1) | | B-2 Dry severe |
| 3 | Oily | (0,1) | 2 | (0,1) | (0,1) | (0,1) | (0,1) | C-Oily | C-1 Oily mild |
| | | (0,1) | 3 | (0,1) | (0,1) | (0,1) | (0,1) | | C-2 Oil severe |
| 4 | Sensitiv e | (0,1) | (0,1) | 2 | (0,1) | (0,1) | (0,1) | D-Sensitive | D-1 Sensitive mild |
| | | (0,1) | (0,1) | 3 | (0,1) | (0,1) | (0,1) | | D-2 Sensitive severe |
| 5 | Atopic | 2 | (0,1) | 2 | (0,1) | (0,1) | (0,1) | E-Atopic | E-1 Atopic mild |
| | | 2 | (0,1) | 3 | (0,1) | (0,1) | (0,1) | | E-2 Atopic moderate |
| | | 3 | (0,1) | 2 | (0,1) | (0,1) | (0,1) | | E-3 Atopic moderate |
| | | 3 | (0,1) | 3 | (0,1) | (0,1) | (0,1) | | E-4 Atopic severe |
| 6 | Seborrh eic | (0,1) | 2 | 2 | (0,1) | (0,1) | (0,1) | F-Seborrheic | F-1 Seborrheic mild |
| | | (0,1) | 2 | 3 | (0,1) | (0,1) | (0,1) | | F-2 Seborrheic moderate |
| | | (0,1) | 3 | 2 | (0,1) | (0,1) | (0,1) | | F-3 Seborrheic moderate |
| | | (0,1) | 3 | 3 | (0,1) | (0,1) | (0,1) | | F-4 Seborrheic severe |
| 7 | Trouble some | (0,1) | (0,1) | (0,1) | 2 | (0,1) | (0,1) | G-Troublesom e | G-1 Troublesome mild |
| | | (0,1) | (0,1) | (0,1) | 3 | (0,1) | (0,1) | | G-2 Troublesome severe |
| 8 | Dry dandruf fy | 0 | (0,1) | (0,1) | (0,1) | (2,3) | (0,1) | H-Dry dandruffy | H-1 Dry dandruffy mild |
| | | 1 | (0,1) | (0,1) | (0,1) | (2,3) | (0,1) | | |
| | | 2 | (0,1) | (0,1) | (0,1) | (2,3) | (0,1) | | H-2 Dry dandruffy severe |
| | | 3 | (0,1) | (0,1) | (0,1) | (2,3) | (0,1) | | |
| 9 | Oily dandruf fy | (0,1) | 1 | (0,1) | (0,1) | (2,3) | (0,1) | I-Oily dandruffy | I-1 Oily dandruffy mild |
| | | (0,1) | 2 | (0,1) | (0,1) | (2,3) | (0,1) | | I-2 Oily dandruffy severe |
| | | (0,1) | 3 | (0,1) | (0,1) | (2,3) | (0,1) | | I-3 Oily dandruffy severe |
| 10 | Alopeci a | (0,1) | (0,1) | (0,1) | (0,1) | (0,1) | 2 | J-Alopecia | J-1 Alopecia mild |
| | | (0,1) | (0,1) | (0,1) | (0,1) | (0,1) | 3 | | J-2 Alopecia severe |

* Classification of artificial intelligence (AI) scalp diagnosis scalp type based on scalp state information (detailed)

**[Table 3]**

| |
|---|
| □ Customized shampoo based on scalp diagnosis results: 24 types |
| □ Customized serum based on scalp diagnosis results: 24 types |

* Defining and confirming 24 types of customized scalp shampoo and 24 types of customized scalp serum which may be recommended according to user's detailed scalp type

**[Table 4]**

| **No** | **Classif ication** | **Scalp image AI analysis** | | | | | | **Prescription matching** | | |
|---|---|---|---|---|---|---|---|---|---|---|
| | | **Micro keratin** | **Excessiv e sebum** | **Erythem a between hair follicles** | **Follicul ar erythe ma/pus tules** | **Dandru ffy** | **Hair loss** | **Prescript ion** | **Shampoo prescription** | **Serum prescription** |
| 1 | Good | (0,1) | (0,1) | (0,1) | (0,1) | (0,1) | (0,1) | A-Good | SA-1 Good | EA-2 Good |
| 2 | Dry | 2 | (0,1) | (0,1) | (0,1) | (0,1) | (0,1) | B-Dry | SB-1 Dry mild | EB-2 Dry mild |
| | | 3 | (0,1) | (0,1) | (0,1) | (0,1) | (0,1) | | SB-2 Dry severe | EB-3 Dry severe |
| 3 | Oily | (0,1) | 2 | (0,1) | (0,1) | (0,1) | (0,1) | C-Oily | SC-1 Oily mild | EC-2 Oily mild |
| | | (0,1) | 3 | (0,1) | (0,1) | (0,1) | (0,1) | | SC-2 Oily severe | EC-3 Oily severe |
| 4 | Sensiti ve | (0,1) | (0,1) | 2 | (0,1) | (0,1) | (0,1) | D-Sensitive | SD-1 Sensitive mild | ED-2 Sensitive mild |
| | | (0,1) | (0,1) | 3 | (0,1) | (0,1) | (0,1) | | SD-2 Sensitive severe | ED-3 Sensitive severe |
| 5 | Atopic | 2 | (0,1) | 2 | (0,1) | (0,1) | (0,1) | E-Atopic | SE-1 Atopic mild | EE-2 Atopic mild |
| | | 2 | (0,1) | 3 | (0,1) | (0,1) | (0,1) | | SE-2 Atopic moderate | EE-2 Atopic moderate |
| | | 3 | (0,1) | 2 | (0,1) | (0,1) | (0,1) | | SE-3 Atopic moderate | EE-3 Atopic moderate |
| | | 3 | (0,1) | 3 | (0,1) | (0,1) | (0,1) | | SE-4 Atopic severe | EE-5 Atopic severe |
| 6 | Seborr heic | (0,1) | 2 | 2 | (0,1) | (0,1) | (0,1) | F-Seborrhei c | SF-1 Seborrheic mild | EF-2 Seborrheic mild |
| | | (0,1) | 2 | 3 | (0,1) | (0,1) | (0,1) | | SF-2 Seborrheic moderate | EF-2 Seborrheic moderate |
| | | (0,1) | 3 | 2 | (0,1) | (0,1) | (0,1) | | SF-3 Seborrheic moderate | EF-3 Seborrheic moderate |
| | | (0,1) | 3 | 3 | (0,1) | (0,1) | (0,1) | | SF-4 Seborrheic severe | EF-5 Seborrheic severe |
| 7 | Trouble some | (0,1) | (0,1) | (0,1) | 2 | (0,1) | (0,1) | G-Troubleso me | SG-1 Troublesome mild | EG-2 Troublesome mild |
| | | (0,1) | (0,1) | (0,1) | 3 | (0,1) | (0,1) | | SG-2 Troublesome severe | EG-3 Troublesome severe |
| 8 | Dry dandru ffy | 0 | (0,1) | (0,1) | (0,1) | (2,3) | (0,1) | H-Dry dandruffy | SH-1 Dry dandruffy mild | EH-2 Dry dandruffy mild |
| | | 1 | (0,1) | (0,1) | (0,1) | (2,3) | (0,1) | | | |
| | | 2 | (0,1) | (0,1) | (0,1) | (2,3) | (0,1) | | SH-2 Dry dandruffy severe | EH-3 Dry dandruffy severe |
| | | 3 | (0,1) | (0,1) | (0,1) | (2,3) | (0,1) | | | |
| 9 | Oily dandru ffy | (0,1) | 1 | (0,1) | (0,1) | (2,3) | (0,1) | I-Oily dandruffy | SI-1 Oily dandruffy mild | EI-2 Oily dandruffy mild |
| | | (0,1) | 2 | (0,1) | (0,1) | (2,3) | (0,1) | | SI-2 Oily dandruffy severe | EI-2 Oily dandruffy severe |
| | | (0,1) | 3 | (0,1) | (0,1) | (2,3) | (0,1) | | SI-2 Oily dandruffy severe | EI-2 Oily dandruffy severe |
| 10 | Alopeci a | (0,1) | (0,1) | (0,1) | (0,1) | (0,1) | 2 | J-Alopecia | SJ-1 Alopecia mild | EJ-2 Alopecia mild |
| | | (0,1) | (0,1) | (0,1) | (0,1) | (0,1) | 3 | | SJ-2 Alopecia severe | EJ-3 Alopecia severe |

* Recommendation of product (shampoo/serum) according to classification of artificial intelligence (AI) scalp diagnosis and scalp type based on scalp state

**[Table 3]**

| **Product** | **Seborrheic scalp** | | |
|---|---|---|---|
| | **Scalp type F-1** | **Scalp type F-2** | **Scalp type F-3** |
| | Scalp with some oil and erythema between hair follicles | Scalp with oil identified even with the naked eye and severe erythema between the hair follicles | Scalp with severe oil and erythema between hair follicles |

| **Shampoo** | **SF-1** | **SF-2** | **SF-3** |
|---|---|---|---|
| | - Strong detergency/bubbling power Amino-based anion-based surfactant | - Strong detergency/bubbling power Amino-based anion-based surfactant Minimized scalp irritation | - Strong detergency/bubbling power Amino-based anion-based surfactant Minimized scalp irritation |
| | Minimized scalp irritation | - Added inflammation function | - Added inflammation function |

| **Serum** | **EF-1** | **EF-2** | **EF-3** |
|---|---|---|---|
| | - Minimized scalp irritation | - Minimized scalp irritation | - Minimized scalp irritation |
| | | - Normalized sebum secretion | - Normalized sebum secretion |
| | - Normalized sebum secretion | | |
| | | - Alleviated scalp irritation | - Alleviated scalp irritation |
| | - Alleviated scalp irritation | - Added antiinflammatory/antibacterial function | - Added antiinflammatory/antibacterial function |

| | | | |
|---|---|---|---|
| * Example of recommendation of seborrheic scalp type product | | | |

Then, as a second embodiment of the present invention, as shown in FIGS. 12 and 13, there may be provided an artificial intelligence processor 5-1 configured to perform AI analysis, which receives a scalp image obtained from a subject by one of a scalp diagnosis device or a terminal 1 through API (RESTful) as a cloud service, and classifies the received scalp image into all or some of diagnosis items (for example, some of ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia).

There may be provided a scalp diagnosis artificial intelligence algorithm 6-1 configured to receive information classified into all or some of the diagnostic items from the AI processor 5-1 and perform learning and reading by a deep learning algorithm to perform a specific precision diagnosis, thereby deriving a final result diagnosis.

The scalp image may be classified into 10 scalp types based on seven pieces of scalp state information by utilizing the accumulated data of the database through the artificial intelligence processor 5-1.

The seven pieces of "scalp state information" may include ① micro keratin, ② excessive sebum, ③ erythema between hair follicles, ④ follicular erythema, ⑤ pustules, ⑥ dandruff, and ⑦ hair loss (low hair density or small hair thickness), and
the 10 "scalp types" may include ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia.

The scalp image may be classified into the following 10 "scalp types" based on the seven pieces of "scalp state information" by an artificial intelligence processor:
(1) good: micro keratin (X), excessive sebum (X), erythema between hair follicles (X), follicular erythema (X) or pustules (X), dandruff (X), hair loss (X);
(2) dry: micro keratin (O), excessive sebum (X), erythema between hair follicles (X), follicular erythema (X) or pustules (X), dandruff (X), hair loss (X);
(3) oily: micro keratin (X), excessive sebum (O), erythema between hair follicles (X), follicular erythema (X) or pustules (X), dandruff (X), hair loss (X);
(4) sensitive: micro keratin (X), excessive sebum (X), erythema between hair follicles (O), follicular erythema (X) or pustules (X), dandruff (X), hair loss (X);
(5) atopic: micro keratin (O), excessive sebum (X), erythema between hair follicles (O), follicular erythema (X) or pustules (X), dandruff (X), hair loss (X);
(6) seborrheic: micro keratin (X), excessive sebum (O), erythema between hair follicles (O), follicular erythema (X) or pustules (X), dandruff (X), hair loss (X);
(7) troublesome (inflammatory): micro keratin (X), excessive sebum (X), erythema between hair follicles (X), follicular erythema (O) or pustules (O), dandruff (X), hair loss (X);
(8) dry dandruffy: micro keratin (O), excessive sebum (X), erythema between hair follicles (X), follicular erythema (X) or pustules (X), dandruff (O), hair loss (X);
(9) oily dandruffy: micro keratin (X), excessive sebum (O), erythema between hair follicles (X), follicular erythema (X) or pustules (X), dandruff (O), hair loss (X); and
(10) alopecia: micro keratin (X), excessive sebum (X), erythema between hair follicles (X), follicular erythema (X) or pustules (X), dandruff (X), hair loss (O), and
the scalp state information may be classified into ① micro keratin, ② excessive sebum, ③ erythema between hair follicles, ④ follicular erythema, ⑤ pustules, ⑥ dandruff, and ⑦ hair loss, may be divided into three stages of 0 (none), 1 (mild), 2 (moderate), and 3 (severe) depending on severity, and may be classified into a scalp type of a single symptom and all the scalp types of combination symptoms (① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia).

A sequence of improving the scalp according to the scalp type may be performed in an order of ① troublesome, ② seborrheic, ③ atopic, ④ oily dandruffy, ⑤ dry dandruffy, ⑥ sensitive, ⑦ alopecia, ⑧ oily, ⑨ dry, ⑩ good, and the sequence of improving may be sequentially performed for all or some of them.

The artificial intelligence processor 5-1 may learn the scalp and collect data as a deep learning step for scalp classification by artificial intelligence (AI) analysis by utilizing information of big data on the received scalp image, may perform labeling for the collected learning data, may perform classification learning and verification of the collected data with learning data and test data (8:2), and may derive an inference model (convolutional neural network: CNN).

For example, as a method for developing an inference model, TensorFlow, which is a deep learning library as a way for development using PyThon, may be used to classify the scalp (CNN: object recognition) through EfficientNet model re-training, and an inference model may be made through transfer learning with the EfficientNet model to make a comparison and make an interference with optimal accuracy, and thus the scalp state information and severity may be analyzed as a whole or in part (for example, ① micro keratin, ② excessive sebum, ③ erythema between hair follicles, ④ follicular erythema, ⑤ pustules, ⑥ dandruff, and ⑦ hair loss).

The EfficientNet model (see FIGS. 8 to 11) may include MBConv as a basic block of the EfficientNet.
- The MBConv may include general Conv layers for performing Depthwise Conv*, Squeeze excitation*, and width scaling up.
- Based on an EfficientNet b0 model, a total number of Conv blocks may be 18, two blocks at both ends may be Conv, and the rest may be MBConv.
- The entire Conv block may be divided into nine parts by combining the repeated blocks.

Stochastic depth* may be applied only to the first MBConv of ③④⑤⑦ after width scaling up.

* Depthwise convolution: One of the grouped convolutions as a convolution technique which combines results by applying different 2D filters for each channel.

A number of parameters may be reduced, and thus there may be an advantage of reducing an amount of computation and increasing a speed.

* Stochastic depth: Dropout of a channel unit.

* Squeeze excitation: After one value per channel is extracted with an AdaptiveAvgPool2d, the extracted value may be passed through a conv filter to reduce a number of channels and restore a number of channels. Then, an input image may be multiplied by a value passed through a sigmoid. This may be equal to considering the importance for each channel.

The scalp diagnosis AI algorithm 6-1 may be configured to receive a diagnosed scalp image from the artificial intelligence processor 5-1, perform learning and reading for the received classification information by using the EfficientNet model (see FIG. 8) as a deep learning algorithm utilizing information of big data of the database, infer an image through an additional retraining of a scalp image set to analyze the scalp state information and resulting severity into seven categories (① micro keratin, ② excessive sebum, ③ erythema between hair follicles, ④ follicular erythema, ⑤ pustules, ⑥ dandruff, and ⑦ hair loss), perform a precision diagnosis on the resulting scalp type for all or some of items: ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia by the scalp diagnosis AI algorithm to derive a final result diagnosis.

The diagnosis method may be a method using an EfficientNet deep learning model, but is not limited thereto, and a Google deep learning model or other deep learning models may be also used.

The derived final result diagnosis may be transmitted to a subject (user) through the API (RESTful) 2 as a cloud service.

According to the present invention, the measured scalp questionnaire data and the scalp image may be transmitted and stored in the server, and the stored scalp questionnaire data and the scalp image may be shared in the server for a recommendation service and the artificial intelligence (AI) server to diagnose and analyze the scalp questionnaire data and the scalp image, thereby maximizing speed and efficiency, accurately analyzing scalp state information, diagnosing a scalp type based on the analysis, implementing a method for improving the scalp according to the diagnosed scalp type, and collecting appropriate improvement methods and products according to the scalp type.

### [Industrial Applicability]

According to the present invention, the measured scalp questionnaire data and the scalp image may be transmitted and stored in the server, and the stored scalp questionnaire data and the scalp image may be shared in the server for a recommendation service and the artificial intelligence server to diagnose and analyze the scalp questionnaire data and the scalp image, thereby maximizing speed and efficiency, accurately analyzing scalp state information, diagnosing a scalp type based on the analysis, implementing a method for improving the scalp according to the diagnosed scalp type, and collecting appropriate products according to the scalp type.

## Claims

1. A scalp type diagnostic system on a basis of scalp state information, the system comprising:
a server (S) for file storage, configured to transmit questionnaire information obtained through a questionnaire answered by a subject as well as a scalp image obtained by one of a scalp diagnosis device or a terminal (1) through API (RESTful) (2) as a cloud service and store a same in file;
a main processor (3) configured to diagnose the questionnaire information through a self algorithm among the questionnaire information directly received from the server (S) for file storage or received through the API (RESTful) (2) as the cloud service as well as the scalp images, analyze the scalp image for all or some of diagnosis items (for example, ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia) by artificial intelligence (AI) analysis of an artificial intelligence processor by utilizing information of big data constructed in database (4), and transmit diagnosis results from the analysis and diagnosis based on the questionnaire information in real time to a terminal of a diagnostician again through the API together with a recommended product in accordance with an appropriate prescription;
an artificial intelligence processor (5) configured to perform AI analysis, which classifies the scalp image directly received from the server for file storage or received from the main processor into all or some of the diagnosis items (for example, some of ① good (normal), ② dry, ③ oily, ④ sensitive, ⑤ atopic, ⑥ seborrheic, ⑦ troublesome, ⑧ dry dandruffy, ⑨ oily dandruffy, ⑩ alopecia) by utilizing accumulated data of database;
a scalp diagnosis AI algorithm (6) configured to receive information classified into all or some of the diagnostic items from the artificial intelligence processor and perform learning and reading by a deep learning algorithm to perform a specific precision diagnosis, thereby deriving a final result diagnosis; and
database (4) configured to accumulate scalp measurement, diagnosis, and recommendation data to be provided to the main processor so as to perform self scalp diagnosis and recommendation service,
wherein the scalp image is classified into 10 scalp types based on seven pieces of scalp state information by utilizing the accumulated data of the database through the artificial intelligence processor (5),
the seven pieces of "scalp state information" includes ① micro keratin, ② excessive sebum, ③ erythema between hair follicles, ④ follicular erythema, ⑤ pustules, ⑥ dandruff, and ⑦ hair loss (low hair density or small hair thickness), and
the 10 "scalp types" include ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia.

2. The scalp type diagnostic system of claim 1, wherein a scalp image is classified into following 10 "scalp types" based on seven pieces of "scalp state information" by an artificial intelligence processor:
(1) good: micro keratin (X), excessive sebum (X), erythema between hair follicles (X), follicular erythema (X) or pustules (X), dandruff (X), hair loss (X);
(2) dry: micro keratin (O), excessive sebum (X), erythema between hair follicles (X), follicular erythema (X) or pustules (X), dandruff (X), hair loss (X);
(3) oily: micro keratin (X), excessive sebum (O), erythema between hair follicles (X), follicular erythema (X) or pustules (X), dandruff (X), hair loss (X);
(4) sensitive: micro keratin (X), excessive sebum (X), erythema between hair follicles (O), follicular erythema (X) or pustules (X), dandruff (X), hair loss (X);
(5) atopic: micro keratin (O), excessive sebum (X), erythema between hair follicles (O), follicular erythema (X) or pustules (X), dandruff (X), hair loss (X);
(6) seborrheic: micro keratin (X), excessive sebum (O), erythema between hair follicles (O), follicular erythema (X) or pustules (X), dandruff (X), hair loss (X);
(7) troublesome (inflammatory): micro keratin (X), excessive sebum (X), erythema between hair follicles (X), follicular erythema (O) or pustules (O), dandruff (X), hair loss (X);
(8) dry dandruffy: micro keratin (O), excessive sebum (X), erythema between hair follicles (X), follicular erythema (X) or pustules (X), dandruff (O), hair loss (X);
(9) oily dandruffy: micro keratin (X), excessive sebum (O), erythema between hair follicles (X), follicular erythema (X) or pustules (X), dandruff (O), hair loss (X); and
(10) alopecia: micro keratin (X), excessive sebum (X), erythema between hair follicles (X), follicular erythema (X) or pustules (X), dandruff (X), hair loss (O).

3. The scalp type diagnostic system of claim 1 or 2, wherein the scalp state information is classified into ① micro keratin, ② excessive sebum, ③ erythema between hair follicles, ④ follicular erythema, ⑤ pustules, ⑥ dandruff, and ⑦ hair loss, is divided into three stages of 0 (none), 1 (mild), 2 (moderate), and 3 (severe) depending on severity, and is classified into a scalp type of a single symptom and all scalp types of combination symptoms (① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia).

4. The scalp type diagnostic system of claim 1, wherein a sequence of improving the scalp according to the scalp type is performed in an order of ① troublesome, ② seborrheic, ③ atopic, ④ oily dandruffy, ⑤ dry dandruffy, ⑥ sensitive, ⑦ alopecia, ⑧ oily, ⑨ dry, ⑩ good, and the sequence of improving is sequentially performed for all or some of them.

5. The scalp type diagnostic system of claim 1, wherein the main processor (3) is configured to diagnose the questionnaire information through a self algorithm constructed in the main processor (3) among the questionnaire information received from a subject as well as the scalp image, analyze the scalp image through artificial intelligence (AI) analysis by utilizing information of big data constructed in database, thereby analyzing the scalp state information and severity into seven categories of ① micro keratin, ② excessive sebum, ③ erythema between hair follicles, ④ follicular erythema, ⑤ pustules, ⑥ dandruff, and ⑦ hair loss, analyze resulting scalp types into some of ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia, and transmit diagnosis results from the analysis and diagnosis based on the questionnaire information in real time to a terminal of a diagnostician again through the API together with a recommended product in accordance with an appropriate prescription.

6. The scalp type diagnostic system of claim 1, wherein the artificial intelligence processor (5), which is designed for a deep learning step for classifying the scalp by artificial intelligence (AI) analysis by utilizing information of big data with regard to the received scalp image, is configured to learn the scalp to collect data, perform labeling for the collected learning data, perform classification learning and verification for the collected data with learning data and test data, and derive an inference model (convolutional neural network: CNN), classify the scalp (CNN: object recognition) through EfficientNet model by using TensorFlow, a deep learning library, as a development using Python as a method of developing an inference model, create an interference model through transfer learning by EfficientNet model to make a comparison and make an inference with optimal accuracy, thereby analyzing the scalp state information and severity for all or some (① micro keratin, ② excessive sebum, ③ erythema between hair follicles, ④ follicular erythema, ⑤ pustules, ⑥ dandruff, and ⑦ hair loss).

7. The scalp type diagnostic system of claim 1, wherein the scalp diagnosis AI algorithm (6) is configured to receive the diagnosed scalp image from the artificial intelligence processor (5), perform learning and reading for the received classification information by using the EfficientNet model as a deep learning algorithm utilizing information of big data of the database, infer an image through an additional retraining of a scalp image set to analyze the scalp state information and resulting severity into seven categories (① micro keratin, ② excessive sebum, ③ erythema between hair follicles, ④ follicular erythema, ⑤ pustules, ⑥ dandruff, and ⑦ hair loss), and perform a precision diagnosis for all or some of items: ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia to derive a final result diagnosis.

8. The scalp type diagnostic system of claim 6 or 7, wherein the EfficientNet model comprises MBConv as a basic block of the EfficientNet and the MBConv comprises convolution layers for performing depthwise convolution, squeeze excitation, and width scaling up.

9. A scalp type diagnostic system on a basis of scalp state information, the system comprising:
an artificial intelligence processor (5-1) configured to perform AI analysis, which receives a scalp image obtained from a subject by one of a scalp diagnosis device or a terminal (1) through API (RESTful) (2) as a cloud service and classifies the received scalp image for all or some of diagnosis items (for example, some of ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia); and
a scalp diagnosis AI algorithm (6-1) configured to receive information classified into all or some of the diagnosis items from the artificial intelligence processor and perform learning and reading by a deep learning algorithm to perform a specific precision diagnosis, thereby deriving a final result diagnosis,
wherein the scalp image is classified into 10 scalp types based on seven pieces of scalp state information by utilizing the accumulated data of the database, the seven pieces of "scalp state information" includes ① micro keratin, ② excessive sebum, ③ erythema between hair follicles, ④ follicular erythema, ⑤ pustules, ⑥ dandruff, and ⑦ hair loss (low hair density or small hair thickness), and the 10 "scalp types" includes ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia.

10. The scalp type diagnostic system of claim 9, wherein the scalp image is classified into following 10 "scalp types" based on seven pieces of "scalp state information" by an artificial intelligence processor:
(1) good: micro keratin (X), excessive sebum (X), erythema between hair follicles (X), follicular erythema (X) or pustules (X), dandruff (X), hair loss (X);
(2) dry: micro keratin (O), excessive sebum (X), erythema between hair follicles (X), follicular erythema (X) or pustules (X), dandruff (X), hair loss (X);
(3) oily: micro keratin (X), excessive sebum (O), erythema between hair follicles (X), follicular erythema (X) or pustules (X), dandruff (X), hair loss (X);
(4) sensitive: micro keratin (X), excessive sebum (X), erythema between hair follicles (O), follicular erythema (X) or pustules (X), dandruff (X), hair loss (X);
(5) atopic: micro keratin (O), excessive sebum (X), erythema between hair follicles (O), follicular erythema (X) or pustules (X), dandruff (X), hair loss (X);
(6) seborrheic: micro keratin (X), excessive sebum (O), erythema between hair follicles (O), follicular erythema (X) or pustules (X), dandruff (X), hair loss (X);
(7) troublesome (inflammatory): micro keratin (X), excessive sebum (X), erythema between hair follicles (X), follicular erythema (O) or pustules (O), dandruff (X), hair loss (X);
(8) dry dandruffy: micro keratin (O), excessive sebum (X), erythema between hair follicles (X), follicular erythema (X) or pustules (X), dandruff (O), hair loss (X);
(9) oily dandruffy: micro keratin (X), excessive sebum (O), erythema between hair follicles (X), follicular erythema (X) or pustules (X), dandruff (O), hair loss (X); and
(10) alopecia: micro keratin (X), excessive sebum (X), erythema between hair follicles (X), follicular erythema (X) or pustules (X), dandruff (X), hair loss (O).

11. The scalp type diagnostic system of claim 9 or 10, wherein the scalp state information is classified into ① micro keratin, ② excessive sebum, ③ erythema between hair follicles, ④ follicular erythema, ⑤ pustules, ⑥ dandruff, and ⑦ hair loss, is divided into three stages of 0 (none), 1 (mild), 2 (moderate), and 3 (severe) depending on severity, and is classified into a scalp type of a single symptom and all scalp types of combination symptoms (① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia).

12. The scalp type diagnostic system of claim 9 to 10, wherein a sequence of improving the scalp according to the scalp type is performed in an order of ① troublesome, ② seborrheic, ③ atopic, ④ oily dandruffy, ⑤ dry dandruffy, ⑥ sensitive, ⑦ alopecia, ⑥ oily, ⑨ dry, ⑩ good, and the sequence of improving is sequentially performed for all or some of them.

13. The scalp type diagnostic system of claim 9, wherein the artificial intelligence processor (5-1), which is designed for a deep learning step for classifying the scalp by artificial intelligence (AI) analysis by utilizing information of big data with regard to the received scalp image, is configured to learn the scalp to collect data, perform labeling for the collected learning data, perform classification learning and verification for the collected data with learning data and test data, and derive an inference model (convolutional neural network: CNN), classify the scalp (CNN: object recognition) through EfficientNet model by using TensorFlow, a deep learning library, as a development using Python as a method of developing an inference model, create an interference model through transfer learning by EfficientNet model to make a comparison and make an inference with optimal accuracy, thereby analyzing the scalp state information and severity for all or some (① micro keratin, ② excessive sebum, ③ erythema between hair follicles, ④ follicular erythema, ⑤ pustules, ⑥ dandruff, and ⑦ hair loss).

14. The scalp type diagnostic system of claim 9, wherein the scalp diagnosis AI algorithm (6-1) is configured to receive the diagnosed scalp image from the artificial intelligence processor (5-1), perform learning and reading for the received classification information by using the EfficientNet model as a deep learning algorithm utilizing information of big data of the database, infer an image through an additional retraining of a scalp image set to analyze the scalp state information and resulting severity into seven categories (① micro keratin, ② excessive sebum, ③ erythema between hair follicles, ④ follicular erythema, ⑤ pustules, ⑥ dandruff, and ⑦ hair loss), and perform a precision diagnosis for all or some of items: ① good (normal), ② dry, ③ oily, ④ sensitive, ⑤ atopic, ⑥ seborrheic, ⑦ troublesome, ⑧ dry dandruffy, ⑨ oily dandruffy, and ⑩ alopecia to derive a final result diagnosis.

15. The scalp type diagnostic system of claim 13 or 14, wherein the EfficientNet model comprises MBConv as a basic block of the EfficientNet and the MBConv comprises convolution layers for performing depthwise convolution, squeeze excitation, and width scaling up.
